# EUROPEAN PATENT APPLICATION

(11) **EP 3 424 557 A1**
(43) Date of publication of application: **09.01.2019**
(21) Application number: 16892738.2
(22) Date of filing: 16.12.2016
(51) Int. Cl.: A61N 1/39, A61N 1/05

(54) **INTRACARDIAC DEFIBRILLATION CATHETER**

(30) Priority: 29.02.2016 JP 2016038472
(71) Applicant: Japan Lifeline Co., Ltd., Tokyo 140-0002 (JP)
(72) Inventor: SASAKI, Takuya, Tokyo 140-0002 (JP)
(74) Representative: Grünecker Patent- und Rechtsanwälte PartG mbB
(86) International application number: PCT/JP2016/087524
(87) International publication number: WO 2017/149904

(57) **Abstract**

An object is to provide a defibrillation catheter that is insertable along a guide wire into a target site in the inside of a cardiac cavity and that can assuredly prevent a short circuit between a first lead wire group and a second lead wire group. The defibrillation catheter of the present invention comprises a multi-lumen tube 10, a first DC electrode group 31G, a second DC electrode group 32G, a first lead wire group 41G made up of lead wires 41 connected to each of constituent electrodes 31 of the first DC electrode group 31G, and a second lead wire group 42G made up of lead wires 42 connected to each of constituent electrodes 32 of the second DC electrode group 32G; and applies voltages between the first DC electrode group 31G and the second DC electrode group 32G. The multi-lumen tube 10 has a center lumen 10L through which the guide wire is insertable, and a sub-lumen 11L and a sub-lumen 12L disposed so as to face each other with the center lumen 10L interposed therebetween. The first lead wire group 41G extends in the sub-lumen 11L, and the second lead wire group 42G extends in the sub-lumen 12L.

## Description

### Technical Field

The present invention relates to an intracardiac defibrillation catheter that is inserted into a cardiac cavity and that eliminates atrial fibrillation.

### Background Art

When atrial fibrillation occurs during cardiac catheterization surgery, electrical defibrillation needs to be performed. The present applicant proposes, as a catheter for performing such defibrillation in the inside of a cardiac cavity, an intracardiac defibrillation catheter comprising an insulating tube member having a multi-lumen structure, a handle connected to a proximal end of the tube member, a first DC electrode group made up of a plurality of ring-shaped electrodes mounted on a distal end region of the tube member, a second DC electrode group made up of a plurality of ring-shaped electrodes spaced apart from the first DC electrode group on a proximal end side and mounted on the tube member, a first lead wire group made up of lead wires connected to each of the electrodes constituting the first DC electrode group, and a second lead wire group made up of lead wires connected to each of the electrodes constituting the second DC electrode group. The first lead wire group and the second lead wire group extend in different lumens of the tube member. When defibrillation is performed, voltages having polarities differing from each other are applied to the first DC electrode group and the second DC electrode group (refer to Patent Literature 1).

By inserting the intracardiac defibrillation catheter of such a structure from a superior vena cava into a right atrium, and by further inserting the intracardiac defibrillation catheter into an opening of a coronary sinus (coronary sinus opening) located at the lower back wall of the right atrium, the intracardiac defibrillation catheter is disposed such that the first DC electrode group is positioned in the inside of the coronary sinus and the second DC electrode group is positioned in the inside of the right atrium, after which voltages having polarities differing from each other are applied to the first DC electrode group and the second DC electrode group. This makes it possible to apply sufficient electrical energy required for defibrillation to the heart undergoing atrial fibrillation.

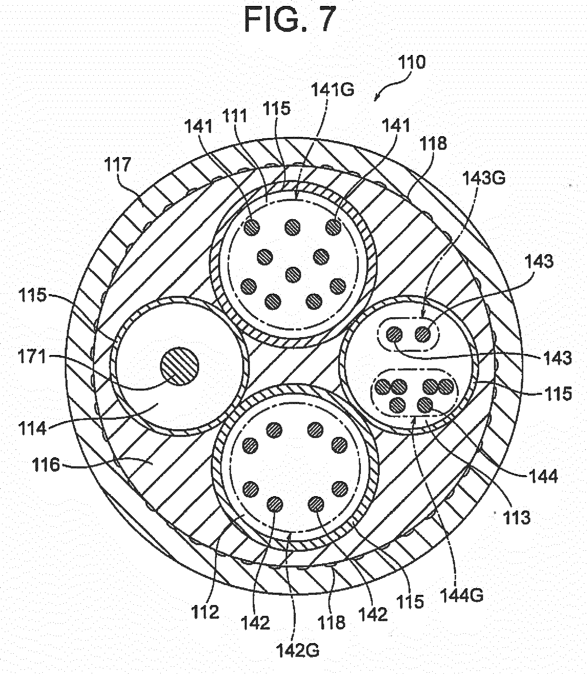

Fig. 7 is a lateral sectional view of the distal end region of the tube member constituting the defibrillation catheter described in Patent Literature 1, and four lumens (a first lumen 111, a second lumen 112, a third lumen 113, and a fourth lumen 114) are formed in this tube member 110.

In Fig. 7, 115 denotes a resin tube that defines the lumen and that is made of fluororesin, 116 denotes an inner part that is made of nylon elastomer having a low hardness, 117 denotes an outer part that is made of nylon elastomer having a high hardness, and 118 denotes a stainless-steel wire rod that forms a braid.

As shown in Fig. 7, a first lead wire group 141G made up of lead wires 141 connected to each of electrodes constituting a first DC electrode group extends in the first lumen 111, a second lead wire group 142G made up of lead wires 142 connected to each of electrodes constituting a second DC electrode group extends in the second lumen 112, a third lead wire group 143G made up of lead wires 143 connected to each of electric-potential-measuring electrodes and a fourth lead wire group 144G made up of lead wires 144 connected to each of electric-potential-measuring electrodes extend in the third lumen 113, and an operating wire 171 extends in the fourth lumen 114.

In this way, since the first lead wire group 141G and the second lead wire group 142G extend in different respective lumens (the first lumen 111 and the second lumen 112) of the tube member, when voltages required for intracardiac defibrillation are applied, it is possible to prevent a short circuit from occurring between the first lead wire group 141G (the first DC electrode group) and the second lead wire group 142G (the second DC electrode group).

### Citation List

### Patent Literature

PTL 1: Japanese Unexamined Patent Application Publication No. 2010-63708

### Summary of Invention

### Technical Problem

The technique of inserting an intracardiac defibrillation catheter from an inferior vena cava into a right atrium and into a coronary sinus opening (an approach from the inferior vena cava) has a lower invasiveness than the conventional technique of inserting an intracardiac defibrillation catheter from a superior vena cava into the right atrium and into the coronary sinus opening (an approach from the superior vena cava).

However, in the approach from the inferior vena cava, the operation of inserting the intracardiac defibrillation catheter into the coronary sinus opening is difficult, and the operation of, for example, even after inserting the intracardiac defibrillation catheter into the coronary sinus opening, applying a torque and reversing a twist of the tube member is required, which is troublesome.

In relation to such a problem, when the approach from the inferior vena cava is performed, inserting the intracardiac defibrillation catheter along a guide wire previously inserted into the coronary sinus opening may be considered.

In order to insert the intracardiac defibrillation catheter along the guide wire into a target site, a lumen (a guide wire lumen) through which the guide wire is insertable needs to be formed in the tube member of the intracardiac defibrillation catheter.

However, the tube member having the guide wire lumen in addition to the lumens in which the lead wires and the operating wire are caused to extend has increased space occupying proportion (the proportion occupied by the resin is reduced), and the wall thickness of the resin separating the lumen in which the first lead wire group is caused to extend and the lumen in which the second lead wire group is caused to extend is reduced. Therefore, when voltages required for defibrillation are applied, a short circuit may occur between the first lead wire group (the first DC electrode group) and the second lead wire group (the second DC electrode group).

Furthermore, the defibrillation catheter comprising the tube member whose space occupying proportion is high (the proportion occupied by the resin is low) due to the tube member having the guide wire lumen has deteriorated torque transmission.

The present invention has been conceived in view of the above circumstances.

A first object of the present invention is to provide an intracardiac defibrillation catheter that is insertable along a guide wire into a target site in the inside of a cardiac cavity and that can assuredly prevent a short circuit between a first lead wire group (a first electrode group) and a second lead wire group (a second electrode group).

A second object of the present invention is to provide an intracardiac defibrillation catheter that can exhibit excellent torque transmission regardless of the proportion of resin of which a tube member is made being low due to the tube member having a guide wire lumen.

### Solution to Problem

(1) An intracardiac defibrillation catheter of the present invention comprises an insulating tube member, a handle connected to a proximal end of the tube member, a first DC electrode group made up of a plurality of ring-shaped electrodes mounted on a distal end region of the tube member, a second DC electrode group made up of a plurality of ring-shaped electrodes spaced apart from the first DC electrode group on a proximal end side and mounted on the distal end region of the tube member, a first lead wire group made up of lead wires connected to each of the electrodes constituting the first DC electrode group, and a second lead wire group made up of lead wires connected to each of the electrodes constituting the second DC electrode group; and performs defibrillation in an inside of a cardiac cavity by applying voltages having polarities differing from each other between the first DC electrode group and the second DC electrode group,
   wherein the tube member is a multi-lumen structural body having a center lumen through which a guide wire is insertable and at least one pair of sub-lumens disposed so as to face each other with the center lumen interposed therebetween, and
   wherein the lead wires constituting the first lead wire group extend in one of the pair of sub-lumens, and the lead wires constituting the second lead wire group extend in the other of the pair of sub-lumens.
   According to the intracardiac defibrillation catheter of such a structure, since the lead wires constituting the first lead wire group and the lead wires constituting the second lead wire group extend in the respective one and other of the pair of sub-lumens disposed so as to face each other with the center lumen interposed therebetween, the lead wires constituting the first lead wire group and the lead wires constituting the second lead wire group can be, in a sufficiently spaced apart state, completely insulated and isolated in the inside of the tube member.
(2) In the intracardiac defibrillation catheter of the present invention, it is preferable that the first lead wire group extend in one of the pair of sub-lumens, and the second lead wire group extend in the other of the pair of sub-lumens.
   According to the intracardiac defibrillation catheter of such a structure, since the first lead wire group (all of the lead wires constituting the first lead wire group) and the second lead wire group (all of the lead wires constituting the second lead wire group) extend in the respective one and other of the pair of sub-lumens disposed so as to face each other with the center lumen interposed therebetween, the first lead wire group and the second lead wire group can be, in a sufficiently spaced apart state, completely insulated and isolated in the inside of the tube member.
(3) In the intracardiac defibrillation catheter of the present invention, it is preferable that the lead wires constituting the first lead wire group be separated and extend in a plurality of sub-lumens, the lead wires constituting the second lead wire group be separated and extend in a plurality of sub-lumens facing each of the plurality of sub-lumens in which the lead wires constituting the first lead wire group extend with the center lumen interposed therebetween, and the lead wires constituting the first lead wire group and the lead wires constituting the second lead wire group do not extend in respective adjacent sub-lumens in a circumferential direction.
   According to the intracardiac defibrillation catheter of such a structure, even when the lead wires constituting the first lead wire group and the lead wires constituting the second lead wire group are separated and extend in the plurality of lumens, since the lead wires constituting the first lead wire group and the lead wires constituting the second lead wire group do not extend in adjacent sub-lumens in the circumferential direction, it is possible to assuredly prevent a short circuit from occurring between the first lead wire group (the first DC electrode group) and the second lead wire group (the second DC electrode group).
(4) In the intracardiac defibrillation catheter of the present invention, it is preferable that the tube member be reinforced by a braid over an entire length thereof and that at least the braid that reinforces the distal end region of the tube member be made of resin.
   According to the intracardiac defibrillation catheter of such a structure, since the tube member is reinforced by the braid over the entire length, excellent torque transmission can be exhibited regardless of the proportion of the resin being low due to the tube member having the center lumen.
   Furthermore, since the braid that reinforces the distal end region of the tube member is made of resin, a side hole for passing the lead wires of the electrodes therethrough can be easily formed in a tube wall of the distal end region of the tube member, and the lead wires inserted through the side hole are not damaged by resin wire rods exposed at an inner peripheral surface of the side hole.
(5) In the intracardiac defibrillation catheter of the aforementioned (4), it is preferable that the tube member be reinforced by the resin braid over the entire length thereof.

According to the intracardiac defibrillation catheter of such a structure, since the tube member is reinforced by the braid over the entire length, excellent torque transmission can be exhibited regardless of the proportion of the resin being low due to the tube member having the center lumen.

Furthermore, since the braid is made of resin, the side hole for passing the lead wires of the electrodes therethrough can be easily formed in the tube wall of the distal end region of the tube member, and the lead wires inserted through the side hole are not damaged by resin wire rods exposed at the inner peripheral surface of the side hole.

Furthermore, since the braid is made of resin, a short circuit does not occur via this braid.

Furthermore, the tube member (the braided tube) reinforced by the resin braid over the entire length can be manufactured by one extrusion molding, and is easily manufactured compared to, for example, a braided tube in which a distal end portion reinforced by a resin braid and a proximal end portion reinforced by a metal braid are joined to each other. Furthermore, kinks that are said to easily occur in a tube portion where a constituent material of the braid is switched do not occur.

### Advantageous Effects of Invention

The intracardiac defibrillation catheter of the present invention is insertable along the guide wire into a target site in the inside of a cardiac cavity, and can easily perform even an operation of inserting the intracardiac defibrillation catheter into a coronary sinus opening by the approach from an inferior vena cava.

Furthermore, according to the intracardiac defibrillation catheter of the present invention, it is possible to assuredly prevent a short circuit from occurring between the first lead wire group (the first DC electrode group) and the second lead wire group (the second DC electrode group).

Furthermore, according to the intracardiac defibrillation catheter of the present invention in which the tube member is reinforced by the resin braid over the entire length, excellent torque transmission can be exhibited regardless of the proportion of the resin of which the tube member is made being low due to the tube member having the center lumen.

### Brief Description of Drawings

Fig. 1 is a plan view showing a defibrillation catheter according to a first embodiment of the present invention.
Fig. 2 is a diagram showing a distal end region of the defibrillation catheter shown in Fig. 1.
Fig. 3A is a lateral sectional view (a sectional view along A-A) of the distal end region of the defibrillation catheter shown in Fig. 2.
Fig. 3B is a lateral sectional view (a sectional view along B-B) of the distal end region of the defibrillation catheter shown in Fig. 2.
Fig. 4 is a plan view showing a defibrillation catheter according to a second embodiment of the present invention.
Fig. 5 is a lateral sectional view (a sectional view along C-C) of a distal end region of the defibrillation catheter shown in Fig. 4.
Fig. 6 is a lateral sectional view showing a distal end region of a defibrillation catheter according to a third embodiment of the present invention.
Fig. 7 is a lateral sectional view showing a distal end portion of a conventional defibrillation catheter. Description of Embodiments

### <First Embodiment>

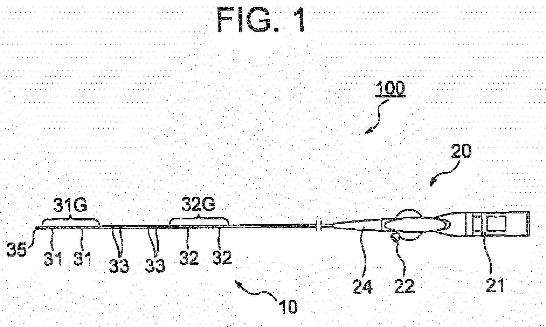

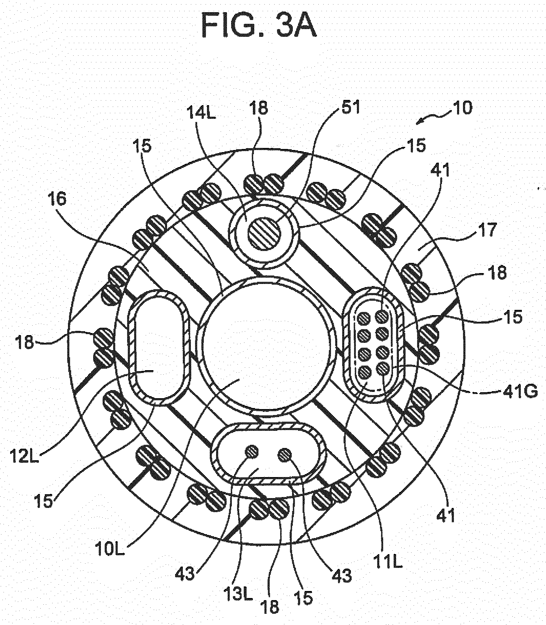

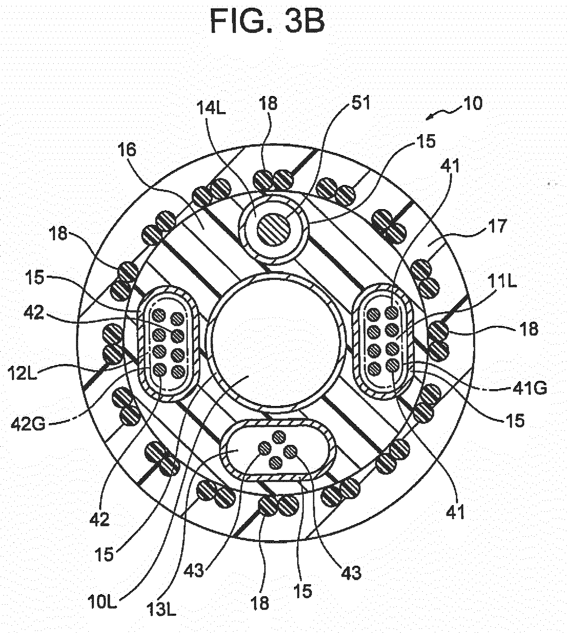

A defibrillation catheter 100 of this embodiment shown in Figs. 1 to 3 (Figs. 3A and 3B) comprises a multi-lumen tube 10, a handle 20 connected to a proximal end of the multi-lumen tube 10, a first DC electrode group 31G made up of eight ring-shaped electrodes 31 mounted on a distal end region of the multi-lumen tube 10, a second DC electrode group 32G made up of eight ring-shaped electrodes 32 spaced apart from the first DC electrode group 31G on a proximal end side and mounted on the distal end region of the multi-lumen tube 10, four electric-potential-measuring ring-shaped electrodes 33 mounted on the distal end region of the multi-lumen tube 10 between the first DC electrode group 31G and the second DC electrode group 32G, a distal end tip 35 mounted on a distal end of the multi-lumen tube 10, a first lead wire group 41G made up of lead wires 41 connected to each of the electrodes 31 constituting the first DC electrode group 31G; a second lead wire group 42G made up of lead wires 42 connected to each of the electrodes 32 constituting the second DC electrode group 32G, and lead wires 43 connected to each of the electric-potential-measuring ring-shaped electrodes 33; and performs defibrillation in the inside of a cardiac cavity by applying voltages having polarities differing from each other between the first DC electrode group 31G and the second DC electrode group 32G. The multi-lumen tube 10 is reinforced by a resin braid 18 over the entire length thereof and has a center lumen 10L, which becomes a guide wire lumen, a first sub-lumen 11L and a second sub-lumen 12L disposed so as to face each other with the center lumen 10L interposed therebetween, and a third sub-lumen 13L and a fourth sub-lumen 14L disposed so as to face each other with the center lumen 10L interposed therebetween. The first lead wire group 41G extends in the first sub-lumen 11L, the second lead wire group 42G extends in the second sub-lumen 12L, and the lead wires 43 extend in the third sub-lumen 13L.

The defibrillation catheter 100 of the present embodiment comprises the multi-lumen tube 10, the handle 20, the first DC electrode group 31G, the second DC electrode group 32G, the electric-potential-measuring ring-shaped electrodes 33, the distal end tip 35, the first lead wire group 41G, the second lead wire group 42G, and the lead wires 43.

The multi-lumen tube 10 constituting the defibrillation catheter 100 is an insulating tube member having a multi-lumen structure.

The outside diameter of the multi-lumen tube 10 is, for example, 1.2 to 3.3 mm, and a preferred example is 2.0 mm.

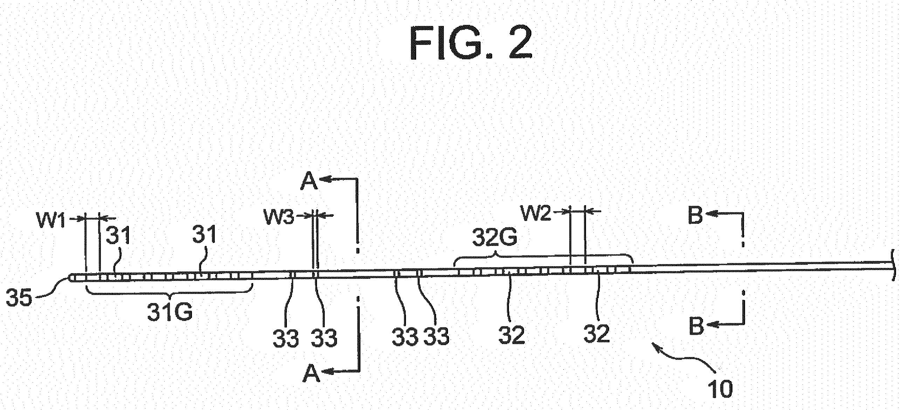

Further, although, in Figs. 1 and 2, the distal end region of the multi-lumen tube 10 is linearly shown, the distal end region ordinarily has a particular curved shape.

As the curved shape of such a distal end region, examples of shapes disclosed in, for example, Japanese Unexamined Patent Application Publication No. 2012-50673 and Japanese Unexamined Patent Application Publication No. 2012-192124 can be given. However, the curved shape is not limited thereto.

Further, the curved shape of such a distal end region is the shape when the distal end region is not subjected to any force from the outside; and the distal end region, for example, is linearly deformed when the multi-lumen tube 10 is passed through a linear tube cavity or is curved in accordance with the shape of a curved tube cavity when the multi-lumen tube 10 is passed through the tube cavity. Furthermore, the shape of the distal end region can be changed by operating the handle 20 described below.

As shown in Figs. 3A and 3B, the multi-lumen tube 10 constituting the defibrillation catheter 100 of this embodiment is a braided tube comprising an inner part 16 made of resin, an outer part 17 that covers the inner part 16 and is made of resin, and the resin braid 18 embedded in an inside portion of the outer part 17 over the entire length of the multi-lumen tube 10.

The center lumen 10L as a guide wire lumen, and the four sub-lumens (the first sub-lumen 11L, the second sub-lumen 12L, the third sub-lumen 13L, and the fourth sub-lumen 14L) around the center lumen 10L are formed in the multi-lumen tube 10 (the inner part 16) by being defined by respective resin tubes 15.

The diameter of the center lumen 10L is, for example, 0.4 to 1.0 mm, and a preferred example is 0.75 mm.

The ratio of the diameter of the center lumen 10L to the outside diameter of the multi-lumen tube 10 is preferably greater than or equal to 0.2, and a preferred example is 0.375 (0.75/2.0).

The first sub-lumen 11L and the second sub-lumen 12L are disposed so as to face each other with the center lumen 10L interposed therebetween.

Furthermore, the third sub-lumen 13L and the fourth sub-lumen 14L are disposed so as to face each other with the center lumen 10L interposed therebetween.

The lateral sections of the first sub-lumen 11L, the second sub-lumen 12L, and the third sub-lumen 13L have respective capsule shapes (oval shapes). This makes it possible to, without increasing the outside diameter of the multi-lumen tube 10 more than is necessary, ensure a wide area of a lumen inner cavity and to simplify the assembly operation.

As the resins of which the inner part 16 and the outer part 17 are made, thermoplastic polyamide elastomer can be mentioned, and, in particular, polyether block amide (PEBAX) is preferable.

The hardness of the resin of which the inner part 16 is made is preferably 25D to 40D.

The hardness of the resin of which the outer part 17 is made is preferably 35D to 72D. Further, for the resin of which the outer part 17 is made, ordinarily, resin having a hardness that differs depending upon an axial direction is used.

The resin tubes 15 that define and form the lumens are made of highly insulating fluororesins, such as perfluoroalkylvinylether copolymer (PFA) and polytetrafluoroethylene (PTFE).

As shown in Figs. 3A and 3B, the resin braid 18, which is a reinforcing material, is embedded in the inside portion of the outer part 17 over the entire length of the multi-lumen tube 10.

In sectional view shown in Figs. 3A and 3B, the braid 18 comprises 16 pairs of resin wire rods (32 pieces) disposed at equal angular intervals in the circumferential direction.

In this way, by reinforcing the multi-lumen tube 10 by the braid 18 over the entire length (the multi-lumen tube 10 is a braided tube), excellent torque transmission can be exhibited regardless of the proportion of the resin being low due to the multi-lumen tube 10 having the center lumen 10L.

Furthermore, since the braid 18 is made of resin, a short circuit does not occur via the braid 18.

Furthermore, since the braid 18 is made of resin, side holes for passing the lead wires (the lead wires 41, 42, and 43) of the electrodes therethrough can be easily formed in a tube wall of the distal end region of the multi-lumen tube 10, and the lead wires inserted through the side holes are not damaged by resin wire rods exposed at inner peripheral surfaces of the side holes.

When a metal braid is embedded instead of the resin braid 18, it becomes difficult to form the side holes for passing the lead wires of the electrodes therethrough in the tube wall of the distal end region of the multi-lumen tube 10, and it is thought that, even if the side holes have been formed, since metal wire rods constituting the braid are exposed at the inner peripheral surfaces of the side holes, when manufacturing and using the defibrillation catheter, the lead wires of the electrodes contact the metal wire rods, resin covering layers constituting the lead wires are damaged, and the insulating characteristic of the lead wires is impaired. Therefore, the metal braid cannot be embedded over the entire length containing the distal end region of the multi-lumen tube 10.

As a constituent material of the braid 18 (the resin wire rods), selection is made from among resins that can exhibit a reinforcing effect by being embedded.

The hardness of the constituent material of the braid 18 is preferably greater than or equal to 72D. When this hardness is too small, a sufficient reinforcing effect, and, thus, good torque transmission may not be exhibited.

Furthermore, the flexural modulus (ISO178 or JIS K7171) of the constituent material of the braid 18 is ordinarily 500 to 19,000 MPa, preferably, 2000 to 7,000 MPa, more preferably, 3,500 to 4,200, and a preferred example is 4,200 MPa.

As a preferred reinforcing resin of which the braid 18 (the resin wire rods) is made, for example, PEEK resin, polyimide resin, polyamide resin, and polyester resin can be mentioned, among which PEEK resin is particularly preferable.

The wire diameter of the resin wire rods constituting the braid 18 is ordinarily 30 to 100 µm, and a preferred example is 60 µm.

Furthermore, the number of carriers of the braid 18 is ordinarily 8 to 32, and a preferred example is 16.

Furthermore, the number of ends of the braid 18 is ordinarily 1 to 4, and a preferred example is 2.

As shown in Fig. 1, the handle 20 constituting the defibrillation catheter 100 of the present embodiment comprises a handle body 21, a knob 22, and a strain relief 24.

By subjecting the knob 22 to a rotating operation, the shape of the distal end region of the multi-lumen tube 10 can be changed.

The first DC electrode group 31G and the second DC electrode group 32G are mounted on an outer periphery of the distal end region of the multi-lumen tube 10.

In the present invention, "electrode group" refers to an assembly of a plurality of electrodes that are mounted at narrow intervals (for example, less than or equal to 5 mm), constituting the same pole (having the same polarity) or having the same purpose.

The first DC electrode group comprises, in the distal end region of the tube member, a plurality of electrodes, constituting the same pole (a minus pole or a plus pole), mounted at narrow intervals. Here, although the number of electrodes constituting the first DC electrode group differs depending upon the electrode widths and arrangement intervals, the number of electrodes is, for example, 4 to 13, and preferably 8 to 10.

In the present embodiment, the first DC electrode group 31G comprises eight ring-shaped electrodes 31. The electrodes 31 constituting the first DC electrode group 31G are connected to a terminal having the same pole in a direct-current power supply device via the lead wires (the lead wires 41 constituting the first lead wire group 41G shown in Figs. 3A and 3B) and a connector built in a proximal end part of the handle 20.

Here, the width (an axial direction length W1) of the electrodes 31 is preferably 2 to 5 mm, and a preferred example is 4 mm.

When the width of the electrodes 31 is too narrow, the heat generation amount when applying voltages becomes too large, and the surrounding tissues may be damaged. On the other hand, when the width of the electrodes 31 is too wide, the flexibility/bendability of a portion where the first DC electrode group 31G has been mounted in the multi-lumen tube 10 may be impaired.

The mounting interval of the electrodes 31 (the separation distance between adjacent electrodes) is preferably 1 to 5 mm, and a preferred example is 2 mm.

When using the intracardiac defibrillation catheter 100 (when the intracardiac defibrillation catheter 100 is disposed in the inside of a cardiac cavity), the first DC electrode group 31G is positioned in the inside of a coronary sinus.

The second DC electrode group comprises, in the distal end region of the tube member spaced apart from the mounting position of the first DC electrode group on a proximal end side, a plurality of electrodes, constituting a pole (a plus pole or a minus pole) opposite to that of the first DC electrode group, mounted at narrow intervals. Here, although the number of electrodes constituting the second DC electrode group differs depending upon the electrode widths and arrangement intervals, the number of electrodes is, for example, 4 to 13, and preferably 8 to 10.

In the present embodiment, the second DC electrode group 32G comprises eight ring-shaped electrodes 32. The electrodes 32 constituting the second DC electrode group 32G are connected to a terminal having the same pole in a direct-current power supply device (a terminal having a pole opposite to that of the terminal to which the first DC electrode group 31G is connected) via the lead wires (the lead wires 42 constituting the second lead wire group 42G shown in Fig. 3B) and a connector built in the proximal end part of the handle 20.

By this, voltages having polarities differing from each other are applied to the first DC electrode group 31G (the electrodes 31) and the second DC electrode group 32G (the electrodes 32), and the first DC electrode group 31G and the second DC electrode group 32G become electrode groups having polarities differing from each other (when one of the electrode groups has a minus pole, the other electrode group has a plus pole).

Here, the width (an axial direction length W2) of the electrodes 32 is preferably 2 to 5 mm, and a preferred example is 4 mm.

When the width of the electrodes 32 is too narrow, the heat generation amount when applying voltages becomes too large, and the surrounding tissues may be damaged. On the other hand, when the width of the electrodes 32 is too wide, the flexibility/bendability of a portion where the second DC electrode group 32G has been mounted in the multi-lumen tube 10 may be impaired.

The mounting interval of the electrodes 32 (the separation distance between adjacent electrodes) is preferably 1 to 5 mm, and a preferred example is 2 mm.

When using the intracardiac defibrillation catheter 100 (when the intracardiac defibrillation catheter 100 is disposed in the inside of a cardiac cavity), the second DC electrode group 32G is positioned in the inside of a right atrium.

Furthermore, the electrodes constituting the first DC electrode group 31G and the second DC electrode group can be used for measuring electric potential.

Four ring-shaped electrodes 33 used in measuring electric potential are mounted on an outer periphery of the multi-lumen tube 10 (between the first DC electrode group 31G and the second DC electrode group 32G).

The electrodes 33 are connected to an electrocardiograph via the lead wires (the lead wires 43 shown in Figs. 3A and 3B) and a connector built in the proximal end part of the handle 20.

Here, the width (an axial direction length W3) of the electrodes 33 is preferably 0.5 to 2.0 mm, and a preferred example is 1.2 mm.

When the width of the electrodes 33 is too wide, the measurement precision of cardiac electric potential is reduced, and it becomes difficult to identify the site of occurrence of abnormal electric potential.

The distal end tip 35 is mounted on a distal end of the intracardiac defibrillation catheter 100.

Lead wires are not connected to this distal end tip 35, and, in the present embodiment, the distal end tip 35 is not used as an electrode. However, by connecting lead wires, the distal end tip 35 can also be used as an electrode. The constituent material of the distal end tip 35 is, for example, a metal material, such as platinum or stainless steel, various resin materials, etc., and is not particularly limited.

The separation distance between the first DC electrode group 31G (the electrodes 31 on the proximal end side) and the second DC electrode group 32G (the electrodes 32 on a distal end side) is preferably 40 to 100 mm, and more preferably 50 to 90 mm.

In order for the electrodes 31 constituting the first DC electrode group 31G, the electrodes 32 constituting the second DC electrode group 32G, and the electric-potential-measuring electrodes 33 to be those providing good contrast imaging with respect to X rays, it is preferable that they be made of platinum or a platinum-based alloy.

The first lead wire group 41G shown in Figs. 3A and 3B is an assembly of eight lead wires 41 connected to each of the eight electrodes 31 constituting the first DC electrode group 31G.

By the first lead wire group 41G (the lead wires 41), each of the eight electrodes 31 constituting the first DC electrode group 31G can be electrically connected to a direct-current power supply device.

The eight electrodes 31 constituting the first DC electrode group 31G are connected to the respective different lead wires 41. Each of the lead wires 41 is, in a distal end portion thereof, welded to an inner peripheral surface of the electrode 31, and enters the first sub-lumen 11L from the side hole formed in the tube wall of the multi-lumen tube 10. The eight lead wires 41 that have entered the first sub-lumen 11L extend, as the first lead wire group 41G, in the first sub-lumen 11L.

The second lead wire group 42G shown in Fig. 3B is an assembly of eight lead wires 42 connected to each of the eight electrodes 32 constituting the second DC electrode group 32G.

By the second lead wire group 42G (the lead wires 42), each of the eight electrodes 32 constituting the second DC electrode group 32G can be electrically connected to a direct-current power supply device.

The eight electrodes 32 constituting the second DC electrode group 32G are connected to the respective different lead wires 42. Each of the lead wires 42 is, in a distal end portion thereof, welded to an inner peripheral surface of the electrode 32, and enters the second sub-lumen 12L from the side hole formed in the tube wall of the multi-lumen tube 10. The eight lead wires 42 that have entered the second sub-lumen 12L extend, as the second lead wire group 42G, in the second sub-lumen 12L.

As described above, since the first lead wire group 41G (the eight lead wires 41) extends in the first sub-lumen 11L, the second lead wire group 42G (the eight lead wires 42) extends in the second sub-lumen 12L, and the first sub-lumen 11L in which the first lead wire group 41G extends and the second sub-lumen 12L in which the second lead wire group 42G extends are disposed so as to face each other with the center lumen 10L interposed therebetween, the separation distance between the first lead wire group 41G and the second lead wire group 42G can be sufficiently ensured.

The four lead wires 43 shown in Figs. 3A and 3B are connected to each of the electric-potential-measuring electrodes 33. By the lead wires 43, each of the electrodes 33 can be connected to an electrocardiograph.

The four electrodes 33 used in measuring electric potential are connected to the respective different lead wires 43. Each of the lead wires 43 is welded to, in a distal end portion thereof, an inner peripheral surface of the electrode 33, and enters the third sub-lumen 13L from the side hole formed in the tube wall of the multi-lumen tube 10 and extends in the third sub-lumen 13L.

The lead wires 41, the lead wires 42, and the lead wires 43 all comprise a resin covering wire in which an outer peripheral surface of a metal conducting wire has been covered by a resin, such as polyimide. Here, the film thickness of the covering resin is on the order of 2 to 30 µm.

In Figs. 3A and 3B, 51 denotes an operating wire.

The operating wire 51 extends in the fourth sub-lumen 14L, and extends so as to be decentered with respect to a center axis of the multi-lumen tube 10.

A distal end portion of the operating wire 51 is, for example, fixed to the distal end tip 35 by soldering.

On the other hand, a proximal end portion of the operating wire 51 is connected to the knob 22 of the handle 20, and the operating wire 51 is pulled by operating the knob 22. By this, the shape of the distal end region of the multi-lumen tube 10 can be changed.

Although the operating wire 51 is made of stainless steel or an Ni-Ti-based superelastic alloy, the operating wire 51 need not necessarily be made of metal. The operating wire 51 may comprise, for example, a nonconducting wire having a high strength.

Further, a mechanism that deflects a distal end part of the multi-lumen tube is not limited thereto, and may comprise, for example, a leaf spring.

Only the operating wire 51 extends in the fourth sub-lumen 14L of the multi-lumen tube 10, and lead wires (lead wire group) do not extend in the fourth sub-lumen 14L. By this, when performing a deflection operation on the distal end part of the multi-lumen tube 10, the lead wires can be prevented from being damaged (for example, abraded) by the operating wire 51 that moves in an axial direction.

In the defibrillation catheter 100 of the present embodiment, even in an inside portion of the handle 20, it is preferable that the first lead wire group 41G (the lead wires 41), the second lead wire group 42G (the lead wires 42), and the lead wires 43 be insulated and isolated.

The defibrillation catheter 100 of the present embodiment is a catheter for performing defibrillation treatment by directly applying electric energy to the heart that fibrillates by applying a direct current voltage between the first DC electrode group 31G and the second DC electrode group 32G.

The defibrillation catheter 100 of the present embodiment is disposed in the inside of a cardiac cavity such that the first DC electrode group 31G is positioned in the inside of a coronary sinus and the second DC electrode group 32G is positioned in the inside of a right atrium. By this, the heart is in a state in which the heart is interposed between the first DC electrode group 31G and the second DC electrode group 32G.

In order for the heart to be disposed in such a state, first, a guide wire is inserted from an inferior vena cava into the right atrium, and is further inserted into the coronary sinus opening located at the lower back wall of the right atrium.

Next, the defibrillation catheter 100 is inserted along this guide wire from the inferior vena cava into the right atrium, is further inserted into the coronary sinus opening located at the lower back wall of the right atrium, and is inserted (advanced) through the coronary sinus opening.

Here, the technique of inserting the defibrillation catheter 100 from the inferior vena cava into the right atrium and into the coronary sinus opening (the approach from the inferior vena cava) has a lower invasiveness than the approach from a superior vena cava, and is thus preferable.

The defibrillation catheter 100 of the present embodiment is suitably used in performing catheterization surgery of the heart that tends to undergo atrial fibrillation. Particularly preferably, cardiac catheterization surgery is performed after previously inserting the intracardiac defibrillation catheter 100 into the cardiac cavity of a patient.

During the cardiac catheterization surgery, when an electrocardiogram measured by constituent electrodes of the first DC electrode group 31G and/or constituent electrodes of the second DC electrode group 32G, or the electric-potential-measuring electrodes 33 is monitored, and atrial fibrillation occurs, the cardiac catheterization surgery is suspended and a defibrillation treatment is performed by the defibrillation catheter 100. Specifically, a direct current voltage is applied between the first DC electrode group 31G and the second DC electrode group 32G via the first lead wire group 41G and the second lead wire group 42G, and electrical energy is directly applied to the heart that fibrillates.

Here, the electrical energy supplied to the heart by the defibrillation catheter 100 is preferably 10 to 30 J.

When the electrical energy is too small, a sufficient defibrillation treatment cannot be performed. On the other hand, when the electrical energy is too large, tissues around the positions of the first DC electrode group 31G and the second DC electrode group 32G may be damaged.

The intracardiac defibrillation catheter 100 of the present embodiment is insertable along the guide wire into a target site in the inside of a cardiac cavity, and can easily perform even an operation of inserting the intracardiac defibrillation catheter into a coronary sinus opening by the approach from an inferior vena cava.

Furthermore, according to this intracardiac defibrillation catheter 100, since the first sub-lumen 11L in which the first lead wire group 41G extends and the second sub-lumen 12L in which the second lead wire group 42G extends are disposed so as to face each other with the center lumen 10L interposed therebetween, the first lead wire group 41G and the second lead wire group 42G can be, in a sufficiently spaced apart state, completely insulated and isolated in the inside of the multi-lumen tube 10. By this, when voltages required for intracardiac defibrillation are applied, it is possible to assuredly prevent a short circuit from occurring between the first lead wire group 41G (the first DC electrode group 31G) and the second lead wire group 42G (the second DC electrode group 32G).

Furthermore, since the multi-lumen tube 10 constituting the intracardiac defibrillation catheter 100 is reinforced by the resin braid 18 over the entire length, excellent torque transmission can be exhibited. Furthermore, since the braid 18 is made of resin, a short circuit does not occur via this braid 18.

### <Second Embodiment>

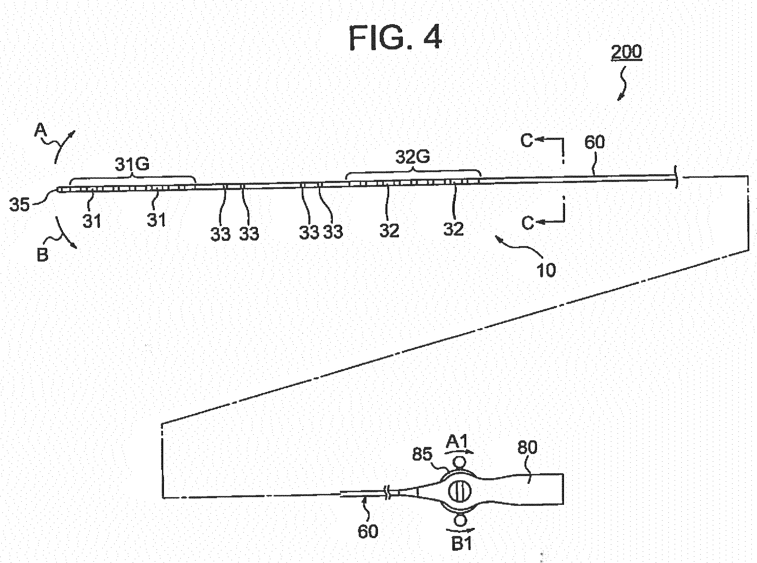

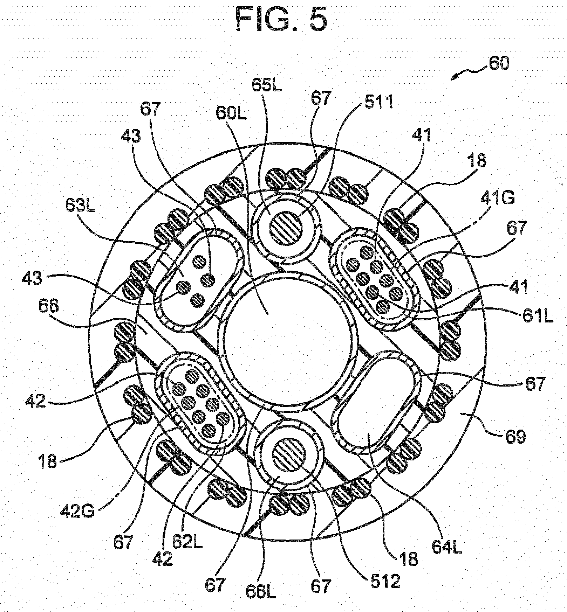

Fig. 4 is a plan view showing a defibrillation catheter 200 of the present embodiment. Fig. 5 is a lateral sectional view (a sectional view along C-C) of a distal end region of this defibrillation catheter 200.

The defibrillation catheter 200 according to the present embodiment comprises a multi-lumen tube 60 having a distal end flexible portion, a control handle 80 connected to a proximal end of the multi-lumen tube 60, a first DC electrode group 31G made up of eight ring-shaped electrodes 31 mounted on the distal end flexible portion of the multi-lumen tube 60, a second DC electrode group 32G made up of eight ring-shaped electrodes 32 spaced apart from the first DC electrode group 31G on a proximal end side and mounted on the distal end flexible portion of the multi-lumen tube 60, four electric-potential-measuring ring-shaped electrodes 33 mounted on the distal end flexible portion of the multi-lumen tube 60 between the first DC electrode group 31G and the second DC electrode group 32G, a distal end tip 35 mounted on a distal end of the multi-lumen tube 60, a first lead wire group 41G made up of lead wires 41 connected to each of the electrodes 31 constituting the first DC electrode group 31G, a second lead wire group 42G made up of lead wires 42 connected to each of the electrodes 32 constituting the second DC electrode group 32G, lead wires 43 connected to each of the electric-potential-measuring ring-shaped electrodes 33, a first operating wire 511 whose proximal end can be subjected to a pulling operation for flexing the distal end flexible portion of the multi-lumen tube 60 in a first direction (a direction shown by an arrow A in Fig. 4), and a second operating wire 512 whose proximal end can be subjected to a pulling operation for flexing the distal end flexible portion of the multi-lumen tube 60 in a second direction (a direction shown by an arrow B in Fig. 4); and performs defibrillation in the inside of a cardiac cavity by applying voltages having polarities differing from each other between the first DC electrode group 31G and the second DC electrode group 32G.

As shown in Fig. 5, the multi-lumen tube 60 constituting the defibrillation catheter 200 of this embodiment is a braided tube comprising an inner part 68 made of resin, an outer part 69 that covers the inner part 68 and is made of resin, and a resin braid 18 embedded in an inside portion of the outer part 69 over the entire length of the multi-lumen tube 60.

A center lumen 60L as a guide wire lumen, and six sub-lumens 61L to 66L around the center lumen 60L are formed in the multi-lumen tube 60 (the inner part 68) by being defined by respective resin tubes 67.

The first sub-lumen 61L and the second sub-lumen 62L are disposed so as to face each other with the center lumen 60L interposed therebetween.

Furthermore, the third sub-lumen 63L and the fourth sub-lumen 64L are disposed so as to face each other with the center lumen 60L interposed therebetween.

Furthermore, the fifth sub-lumen 65L and the sixth sub-lumen 66L are disposed so as to face each other with the center lumen 60L interposed therebetween.

The lateral sections of the first sub-lumen 61L, the second sub-lumen 62L, the third sub-lumen 63L, and the fourth sub-lumen 64L have respective capsule shapes (oval shapes).

Furthermore, the lateral sections of the fifth sub-lumen 65L and the sixth sub-lumen 66L have respective circular shapes.

Furthermore, similarly to the multi-lumen tube 10 constituting the defibrillation catheter 100 according to the first embodiment, the multi-lumen tube 60 is reinforced by the resin braid 18 over the entire length thereof.

The first lead wire group 41G shown in Fig. 5 is an assembly of eight lead wires 41 connected to each of the eight electrodes 31 constituting the first DC electrode group 31G.

By the first lead wire group 41G (the lead wires 41), each of the eight electrodes 31 constituting the first DC electrode group 31G can be electrically connected to a direct-current power supply device.

The eight electrodes 31 constituting the first DC electrode group 31G are connected to the respective different lead wires 41. Each of the lead wires 41 is, in a distal end portion thereof, welded to an inner peripheral surface of the electrode 31, and enters the first sub-lumen 61L from a side hole formed in a tube wall of the multi-lumen tube 60. The eight lead wires 41 that have entered the first sub-lumen 61L extend, as the first lead wire group 41G, in the first sub-lumen 61L.

The second lead wire group 42G shown in Fig. 5 is an assembly of eight lead wires 42 connected to each of the eight electrodes 32 constituting the second DC electrode group 32G.

By the second lead wire group 42G (the lead wires 42), each of the eight electrodes 32 constituting the second DC electrode group 32G can be electrically connected to a direct-current power supply device.

The eight electrodes 32 constituting the second DC electrode group 32G are connected to the respective different lead wires 42. Each of the lead wires 42 is, in a distal end portion thereof, welded to an inner peripheral surface of the electrode 32, and enters the second sub-lumen 62L from a side hole formed in the tube wall of the multi-lumen tube 60. The eight lead wires 42 that have entered the second sub-lumen 62L extend, as the second lead wire group 42G, in the second sub-lumen 62L.

As described above, since the first lead wire group 41G (the eight lead wires 41) extends in the first sub-lumen 61L, the second lead wire group 42G (the eight lead wires 42) extends in the second sub-lumen 62L, and the first sub-lumen 61L in which the first lead wire group 41G extends and the second sub-lumen 62L in which the second lead wire group 42G extends are disposed so as to face each other with the center lumen 60L interposed therebetween, the separation distance between the first lead wire group 41G and the second lead wire group 42G can be sufficiently ensured.

The four lead wires 43 shown in Fig. 5 are connected to each of the electric-potential-measuring electrodes 33. By the lead wires 43, each of the electrodes 33 can be connected to an electrocardiograph.

The four electrodes 33 used in measuring electric potential are connected to the respective different lead wires 43. Each of the lead wires 43 is welded to, in a distal end portion thereof, an inner peripheral surface of the electrode 33, and enters the third sub-lumen 63L from a side hole formed in the tube wall of the multi-lumen tube 60 and extends in the third sub-lumen 63L.

The defibrillation catheter 200 of the present embodiment comprises the first operating wire 511 for flexing the distal end flexible portion of the multi-lumen tube 60 in the first direction (the direction shown by the arrow A), and the second operating wire 512 for flexing this distal end flexible portion in the second direction (the direction shown by the arrow B).

Here, "distal end flexible portion" refers to a distal end region of the multi-lumen tube that can be flexed by a pulling operation of the operating wires (the first operating wire 511 and the second operating wire 512).

The first operating wire 511 is inserted so as to be movable in a tube axis direction in the fifth sub-lumen 65L of the multi-lumen tube 60. A distal end portion of the first operating wire 511 is, for example, connected and fixed to the distal end tip 35 by solder with which an internal space of the distal end tip 35 is filled. Furthermore, the proximal end of the first operating wire 511 is connected to a knob 85 of the control handle 80, and can be subjected to a pulling operation.

On the other hand, the second operating wire 512 is inserted so as to be movable in a tube axis direction in the sixth sub-lumen 66L of the multi-lumen tube 60. A distal end portion of the second operating wire 512 is, for example, connected and fixed to the distal end tip 35 by solder with which the internal space of the distal end tip 35 is filled. Furthermore, the proximal end of the second operating wire 512 is connected to the knob 85 of the control handle 80, and can be subjected to a pulling operation.

When the knob 85 of the control handle 80 is rotated in an A1 direction shown in Fig. 4, the first operating wire 511 is pulled and moves towards a proximal end side of the fifth sub-lumen 65L, and the distal end flexible portion can be flexed in the first direction (the direction shown by the arrow A).

On the other hand, when the knob 85 of the control handle 80 is rotated in a B1 direction shown in Fig. 4, the second operating wire 512 is pulled and moves towards a proximal end side of the sixth sub-lumen 66L, and the distal end flexible portion can be flexed in the second direction (the direction shown by the arrow B).

As shown in Fig. 5, lead wires are not inserted through the fourth sub-lumen 64 of the multi-lumen tube 60, and the fourth sub-lumen 64 is a vacant lumen.

According to the defibrillation catheter 200 of this embodiment, since the first sub-lumen 61L in which the first lead wire group 41G extends and the second sub-lumen 62L in which the second lead wire group 42G extends are disposed so as to face each other with the center lumen 60L interposed therebetween, the first lead wire group 41G and the second lead wire group 42G, in a sufficiently spaced apart state, can be completely insulated and isolated in the inside of the multi-lumen tube 60. By this, when voltages required for intracardiac defibrillation are applied, it is possible to assuredly prevent a short circuit from occurring between the first lead wire group 41G (the first DC electrode group 31G) and the second lead wire group 42G (the second DC electrode group 32G).

### <Third Embodiment>

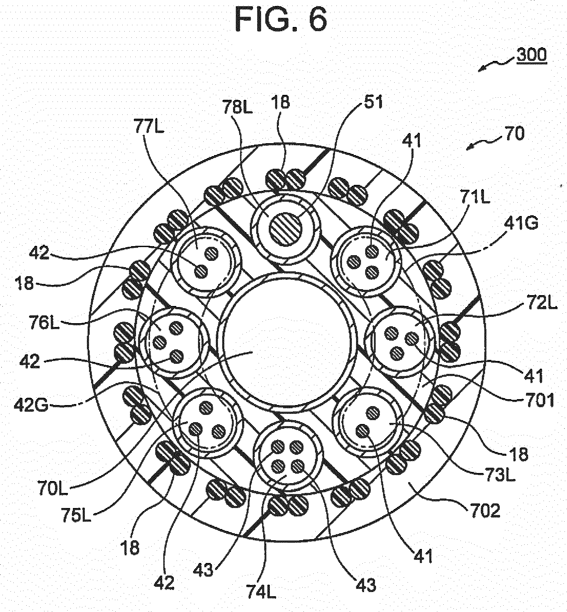

Fig. 6 is a lateral sectional view showing a distal end region of a defibrillation catheter 300 of the present embodiment.

Since the appearance form of this defibrillation catheter 300 is similar to that of the defibrillation catheter 100 according to the first embodiment shown in Figs. 1 and 2, the illustration thereof is omitted. However, the lateral section shown in Fig. 6 is a lateral section at a location that is the same as that in the sectional view along B-B of Fig. 2.

The defibrillation catheter 300 of the present embodiment comprises a multi-lumen tube 70, a handle connected to a proximal end of the multi-lumen tube 70, a first DC electrode group made up of eight ring-shaped electrodes mounted on a distal end region of the multi-lumen tube 70, a second DC electrode group made up of eight ring-shaped electrodes spaced apart from the first DC electrode group on a proximal end side and mounted on the distal end region of the multi-lumen tube 70, four electric-potential-measuring ring-shaped electrodes mounted on the distal end region of the multi-lumen tube 70 between the first DC electrode group and the second DC electrode group, a distal end tip mounted on a distal end of the multi-lumen tube 70, a first lead wire group 41G made up of lead wires 41 connected to each of the electrodes constituting the first DC electrode group; a second lead wire group 42G made up of lead wires 42 connected to each of the electrodes constituting the second DC electrode group, and lead wires 43 connected to each of the electric-potential-measuring ring-shaped electrodes; and performs defibrillation in the inside of a cardiac cavity by applying voltages having polarities differing from each other between the first DC electrode group and the second DC electrode group.

As shown in Fig. 6, the multi-lumen tube 70 constituting the defibrillation catheter 300 of this embodiment is a braided tube comprising an inner part 701 made of resin, an outer part 702 that covers the inner part 701 and is made of resin, and a resin braid 18 embedded in an inside portion of the outer part 702 over the entire length of the multi-lumen tube 70.

A center lumen 70L as a guide wire lumen, and the eight sub-lumens 71L to 78L around the center lumen 10L are formed in the multi-lumen tube 70 (the inner part 701) by being defined by respective resin tubes.

As shown in Fig. 6, the lateral sections of the eight sub-lumens 71L to 78L have respective circular shapes having a diameter that is smaller than that of the center lumen 70L.

Furthermore, similarly to the multi-lumen tube 10 constituting the defibrillation catheter 100 according to the first embodiment, the multi-lumen tube 70 is reinforced by the resin braid 18 over the entire length thereof.

In the multi-lumen tube 70, the first sub-lumen 71L and the fifth sub-lumen 75 are disposed so as to face each other with the center lumen 70L interposed therebetween.

Three lead wires 41 constituting the first lead wire group 41G extend in the first sub-lumen 71L, and three lead wires 42 constituting the second lead wire group 42G extend in the fifth sub-lumen 75L.

Furthermore, the second sub-lumen 72L and the sixth sub-lumen 76L are disposed so as to face each other with the center lumen 70L interposed therebetween.

Three lead wires 41 constituting the first lead wire group 41G extend in the second sub-lumen 72L, and three lead wires 42 constituting the second lead wire group 42G extend in the sixth sub-lumen 76L.

Furthermore, the third sub-lumen 73L and the seventh sub-lumen 77L are disposed so as to face each other with the center lumen 70L interposed therebetween.

Two lead wires 41 constituting the first lead wire group 41G extend in the third sub-lumen 73L, and two lead wires 42 constituting the second lead wire group 42G extend in the seventh sub-lumen 77L.

Furthermore, the fourth sub-lumen 74L and the eighth sub-lumen 78L are disposed so as to face each other with the center lumen 70L interposed therebetween.

Four lead wires 43 connected to each of the electric-potential-measuring ring-shaped electrodes extend in the fourth sub-lumen 74L, and the operating wire 51 extends in the eighth sub-lumen 78L.

According to the defibrillation catheter 300 of this embodiment, the eight lead wires 41 constituting the first lead wire group 41G are separated and extend in the sub-lumens 71L to 73L, the eight lead wires 42 constituting the second lead wire group 42G are separated and extend in the sub-lumens 75L to 77L facing each of the sub-lumens 71L to 73L, the eighth sub-lumen 78L in which the operating wire 51 extends is formed between the first sub-lumen 71L in which the lead wires 41 extend and the seventh sub-lumen 77L in which the lead wires 42 extend, the fourth sub-lumen 74L in which the lead wires 43 extend is formed between the third sub-lumen 73L in which the lead wires 41 extend and the fifth sub-lumen 75L in which the lead wires 42 extend, and the lead wires 41 and the lead wires 42 do not extend in adjacent sub-lumens in a circumferential direction. Therefore, the lead wires 41 constituting the first lead wire group 41G and the lead wires 42 constituting the second lead wire group 42G can be, in a sufficiently spaced apart state, completely insulated and isolated in the inside of the multi-lumen tube 70. By this, when voltages required for intracardiac defibrillation are applied, it is possible to assuredly prevent a short circuit from occurring between the first lead wire group 41G (the first DC electrode group) and the second lead wire group 42G (the second DC electrode group).

Further, as a modification of the present embodiment, by causing the two lead wires 41 extending in the third sub-lumen 73L to extend in the respective first sub-lumen 71L and second sub-lumen 72L, and by causing the two lead wires 42 extending in the seventh sub-lumen 77L to extend in the respective fifth sub-lumen 75L and sixth sub-lumen 76L, the third sub-lumen 73L and the seventh sub-lumen 77L may be vacant lumens.

Although embodiments of the present invention have been described above, the present invention is not limited thereto, and various changes are possible.

For example, a braid that reinforces the distal end region of the multi-lumen tube where electrodes are mounted may be made of resin (a braid similar to the braid 18), and a braid that reinforces a proximal end region of the multi-lumen tube where electrodes are not mounted may be made of metal, such as stainless steel.

### Reference Signs List

- 100: defibrillation catheter
- 10: multi-lumen tube
- 10L: center lumen
- 11L to 14L: sub-lumen
- 15: resin tube
- 16: inner part
- 17: outer part
- 18: braid
- 20: handle
- 21: handle body
- 22: knob
- 24: strain relief
- 31G: first DC electrode group
- 32G: second DC electrode group
- 31, 32, 33: electrode
- 35: distal end tip
- 41G: first lead wire group
- 42G: second lead wire group
- 41, 42, 43: lead wire
- 51: operating wire
- 200: defibrillation catheter
- 60: multi-lumen tube
- 60L: center lumen
- 61L to 66L: sub-lumen
- 68: inner part
- 68: outer part
- 67: resin tube
- 80: control handle
- 85: knob
- 300: defibrillation catheter
- 70: multi-lumen tube
- 70L: center lumen
- 71L to 78L: sub-lumen
- 701: inner part
- 702: outer part

## Claims

1. An intracardiac defibrillation catheter comprising an insulating tube member, a handle connected to a proximal end of the tube member, a first electrode group made up of a plurality of ring-shaped electrodes mounted on a distal end region of the tube member, a second electrode group made up of a plurality of ring-shaped electrodes spaced apart from the first electrode group on a proximal end side and mounted on the distal end region of the tube member, a first lead wire group made up of lead wires connected to each of the electrodes constituting the first electrode group, and a second lead wire group made up of lead wires connected to each of the electrodes constituting the second electrode group; and performing defibrillation in an inside of a cardiac cavity by applying voltages having polarities differing from each other between the first electrode group and the second electrode group,
wherein the tube member is a multi-lumen structural body having a center lumen through which a guide wire is insertable and at least one pair of sub-lumens disposed so as to face each other with the center lumen interposed therebetween, and
wherein the lead wires constituting the first lead wire group extend in one of the pair of sub-lumens, and the lead wires constituting the second lead wire group extend in the other of the pair of sub-lumens.

2. The intracardiac defibrillation catheter according to Claim 1, wherein the first lead wire group extends in one of the pair of sub-lumens, and the second lead wire group extends in the other of the pair of sub-lumens.

3. The intracardiac defibrillation catheter according to Claim 1, wherein the lead wires constituting the first lead wire group are separated and extend in a plurality of sub-lumens, the lead wires constituting the second lead wire group are separated and extend in a plurality of sub-lumens facing each of the plurality of sub-lumens in which the lead wires constituting the first lead wire group extend with the center lumen interposed therebetween, and the lead wires constituting the first lead wire group and the lead wires constituting the second lead wire group do not extend in respective adjacent sub-lumens in a circumferential direction.

4. The intracardiac defibrillation catheter according to any one of Claims 1 to 3, wherein the tube member is reinforced by a braid over an entire length thereof, and at least the braid that reinforces the distal end region of the tube member is made of resin.

5. The intracardiac defibrillation catheter according to Claim 4, wherein the tube member is reinforced by the resin braid over the entire length thereof.
